# EUROPEAN PATENT APPLICATION

(11) **EP 3 988 639 A1**
(43) Date of publication of application: **27.04.2022**
(21) Application number: 20826608.0
(22) Date of filing: 15.06.2020
(51) Int. Cl.: C12M 1/32, C12M 3/00, C12N 5/00, C12N 1/02

(54) **CELL RECOVERY METHOD AND CELL CULTURE DEVICE**

(30) Priority: 20.06.2019 JP 2019114230
(71) Applicant: Sinfonia Technology Co., Ltd., Tokyo 105-8564 (JP)
(72) Inventor: HORII, Daichi, Tokyo 105-8564 (JP); TAKEUCHI, Haruki, Tokyo 105-8564 (JP); SUDA, Yoshimasa, Tokyo 105-8564 (JP); URABE, Yuji, Tokyo 105-8564 (JP)
(74) Representative: Angerhausen, Christoph
(86) International application number: PCT/JP2020/023462
(87) International publication number: WO 2020/255930

(57) **Abstract**

Damage to cells is suppressed in a cell recovery method that does not require a centrifuge. When recovering cells which are cultured in at least one container containing a liquid medium and adhere to an inner surface of the container, a medium discharge step (step S 11) of discharging the medium from the container, a peeling liquid supply step (step S12) of, after the medium is discharged, supplying a peeling liquid for peeling the cells from the inner surface of the container to the container, a peeling liquid discharge step (step S14) of discharging the peeling liquid from the container before the cells are completely peeled from the inner surface of the container, a waiting step (step S15) of, after the peeling liquid is discharged, waiting until the cells are peeled by action of a residual peeling liquid, and a recovery liquid supply step (step S16) of, after the waiting step is completed, supplying a recovery liquid for recovering the cells to the container are performed.

## Description

### TECHNICAL FIELD

The present disclosure relates to a cell recovery method for recovering cells which are cultured in a container containing a liquid medium and adhere to the inner surface of the container, and a cell culture device capable of executing the cell recovery method.

### BACKGROUND

When culturing cells in a container containing a liquid medium, the cells in the container need to be recovered at the time of subculture in which the cells are transferred to another container during a culturing process or at the time of harvesting in which the cells are harvested after the culture is completed. Conventionally, such a cell recovery operation has been performed as follows. That is, after discharging the medium in the container, a peeling liquid is supplied to the container, and the cells adhering to the inner surface of the container are peeled by the action of the peeling liquid. Next, the peeling liquid containing the cells is transferred to a centrifuge tube, and the cells are recovered by separating the cells in the centrifuge tube from the peeling liquid by a centrifuge.

In recent years, the development of a cell culture device that automatically cultures iPS cells and ES cells has been promoted. However, when a centrifuge is introduced into such a cell culture device, a problem is posed in that the device becomes large and the cost increases. In view of this, the cell culture device described in Patent Document 1 adopts a cell recovery method that does not require a centrifuge. Specifically, after the peeling liquid is supplied, the peeling liquid is discharged when the adhesive force of the cells is weakened, and then the medium is supplied into the container. The cells are peeled from the inner surface of the container by the force of the flowing medium at this time. This eliminates the need to separate the cells from the peeling liquid, thereby eliminating the need for a centrifuge.

### [Prior Art Document]

### Patent Document

Patent Document 1: Japanese laid-open publication No. 2017-6058

However, in the cell recovery method described in Patent Document 1, cells having a weak adhesive force are peeled from the inner surface of the container by the force of flowing medium. Therefore, an excessive force is applied to the cells, which may damage the cells. Further, in Patent Document 1, the cells are peeled before the action of the peeling liquid is sufficiently exerted on the cells. Therefore, the peeled cells are in the form of agglomerates. For that reason, after the cells are recovered, the cell agglomerates are disrupted by allowing the medium containing the cells (the cell suspension) to pass through a tube pump. In this step as well, the cells may be damaged by being handled by the tube pump.

The present disclosure considers the above matters, and provides some embodiments of a cell recovery method that does not require a centrifuge and can reduce damage to cells.

### SUMMARY

According to one embodiment of the present disclosure, there is provided a cell recovery method for recovering cells which are cultured in at least one container containing a liquid medium and adhere to an inner surface of the container, the method including performing: a medium discharge step of discharging the liquid medium from the container; after the liquid medium is discharged, a peeling liquid supply step of supplying a peeling liquid for peeling the cells from the inner surface of the container to the container; a peeling liquid discharge step of discharging the peeling liquid from the container before the cells are completely peeled from the inner surface of the container; after the peeling liquid is discharged, a waiting step of waiting until the cells are peeled by action of a residual peeling liquid; and after the waiting step is completed, a recovery liquid supply step of supplying a recovery liquid for recovering the cells to the container.

In the cell recovery method according to the present disclosure, after the peeling liquid is discharged, the recovery liquid is supplied after waiting until the cells are peeled by the action of the residual peeling liquid. Therefore, it is not necessary to separate the cells from the peeling liquid, and it is possible to eliminate the need for a centrifuge. Further, since the process waits until the cells are separated by the action of the residual peeling liquid, it is not necessary to forcibly separate the cells adhering to the inner surface of the container by the force of the flowing recovery liquid. Moreover, since the cells are sufficiently separated from each other by the action of the residual peeling liquid, it is not necessary to disintegrate the cell agglomerates with a tube pump or the like. Therefore, according to the present disclosure, it is possible to suppress damage to the cells while eliminating the need for a centrifuge.

According to another embodiment of the present disclosure, there is provided a cell culture device configured to execute the cell recovery method described above, including: a medium supply/discharge device configured to supply and discharge the liquid medium to and from the container; a peeling liquid supply/discharge device configured to supply and discharge the peeling liquid to and from the container, a recovery liquid supply/discharge device configured to supply and discharge the recovery liquid to and from the container; and a controller, wherein the cell recovery method is executed by controlling, by the controller, operations of the medium supply/discharge device, the peeling liquid supply/discharge device and the recovery liquid supply/discharge device.

According to the cell culture device according to the present disclosure, the above-described cell recovery method can be automatically executed without human intervention.

In the cell culture device according to the present disclosure, the at least one container may include at least two containers, the at least two containers may be connected via a connection path, and the recovery liquid containing the cells in one container of the at least two containers may be transferred to another container of the at least two containers by feeding a gas to the one container of the at least two containers after the cell recovery method is executed in the one container of the at least two containers.

According to such a configuration, during the subculture in which the cell suspension (the recovery liquid containing the cells) is transferred from one container of the at least two containers to another container of the at least two containers, the cell suspension can be transferred without passing through a tube pump or the like. Therefore, it is possible to suppress damage to the cells.

### BRIEF DESCRIPTION OF DRAWINGS

FIG. 1 is a schematic front view showing the configuration of a cell culture device according to an embodiment of the present disclosure.
FIG. 2 is a front view showing the configuration of a culture part.
FIG. 3 is a diagram showing a culture circuit formed inside the cell culture device.
FIG. 4 is a flowchart showing a series of flows of subculture.
FIGS. 5A to 5E are schematic diagrams showing a cell recovery operation.

### DETAILED DESCRIPTION

Embodiments of the present disclosure will now be in detail described in detail with reference to the accompanying drawings.

### (Configuration of Cell Culture Device)

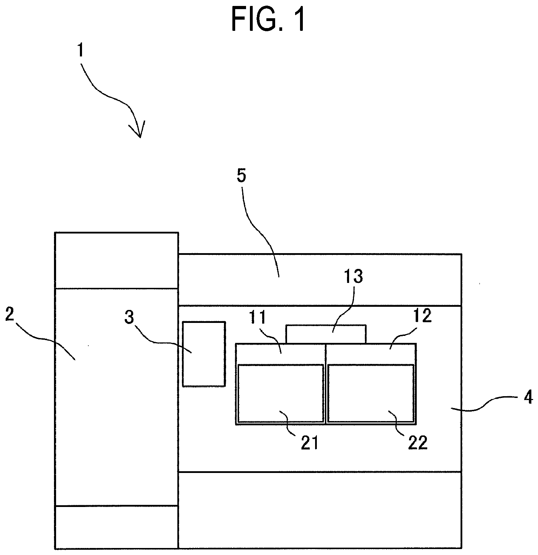

As shown in FIG. 1, the cell culture device 1 according to the present embodiment includes a refrigerating storage part 2, a heater 3, a culture part 4, and a controller 5. The cell culture device 1 is a device for culturing cells according to the data inputted to and stored in the controller 5. In the following description, the front-rear direction is defined as a direction perpendicular to the drawing sheet surface in FIG. 1.

The refrigerating storage part 2 and the heater 3 are housings in which shelves for arranging containers containing media or reagents are formed. Although not shown, the front surfaces of the refrigerating storage part 2 and the heater 3 are provided with doors that can open and close the openings formed on the front surfaces of the housings. The refrigerating storage part 2 is provided with a cooling mechanism (not shown), and the internal temperature thereof is maintained at an arbitrary temperature lower than the room temperature. The heater 3 is arranged inside the culture part 4, and the temperature inside the heater 3 is substantially equal to the temperature inside the culture part 4. Further, tubes are connected to the container arranged inside the refrigerating storage part 2 and the container arranged inside the heater 3 so that the liquids inside the containers can flow out through the tubes. The liquids inside the containers can be discharged by a pump 102 described later. Examples of the container include a bottle, a bag and the like.

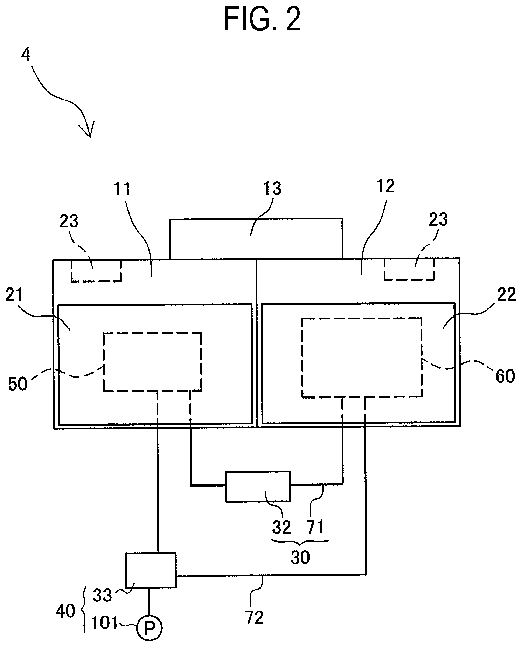

As shown in FIGS. 1 and 2, the culture part 4 includes a first chamber 11 including a first opening/closing part 21 on the front surface thereof, a second chamber 12 including a second opening/closing part 22 on the front surface thereof, and an environment adjustment part 13. The first opening/closing part 21, the second opening/closing part 22, and the wall surfaces of the first chamber 11 and the second chamber 12 are made of a heat insulating material. As a result, the temperatures inside the first chamber 11 and the second chamber 12 are kept constant in a state in which the first opening/closing part 21 and the second opening/closing part 22 are closed. Further, as shown in FIG. 2, a closed container 50 is installed inside the first chamber 11, and a closed container 60 is installed inside the second chamber 12. The inside of the closed containers 50 and 60 is aseptic. Examples of the container include a flask, a multi-layer container, a bag, and the like. Further, in the present embodiment, the closed containers 50 and 60 are made of a material having CO₂ permeability. However, the closed containers 50 and 60 may be made of a material that does not allow CO₂ to pass therethrough. Further, the volume of the closed container 60 is larger than the volume of the closed container 50. This is because when the cells cultured in the closed container 50 and having a high concentration are transferred to the closed container 60 and further cultured in the closed container 60, the amount of the medium contained in the closed container 60 needs to be larger than the amount of the medium contained in the closed container 50. However, it is not essential that the volume of the closed container 60 is larger than the volume of the closed container 50. The volume of the closed container 60 may be smaller than or equal to the volume of the closed container 50. The dotted line portions in FIG. 2 mean that certain components are arranged inside the first chamber 11 and the second chamber 12.

The environment adjustment part 13 includes a built-in heating device and a CO₂ supply device and can adjust the temperatures and CO₂ concentrations inside the first chamber 11 and the second chamber 12 according to a signal sent from the controller 5. Further, sensors 23 for detecting the temperatures and CO₂ concentrations are arranged inside the first chamber 11 and the second chamber 12. The information detected by the sensors 23 is outputted to the controller 5. Devices for adjusting other internal environments may be built in the environment adjustment part 13. In this case, the sensors 23 are sensors that can also detect other internal environments.

Further, as shown in FIG. 2, the culture part 4 includes a connection path 30 for connecting the closed container 50 and the closed container 60 to each other, and a driving part 40 for moving cells between the closed container 50 and the closed container 60 via the connection path 30. The connection path 30 includes a tube 71 and a stirring part 32, the insides of which are kept in an aseptic state. The stirring part 32 is connected to the closed containers 50 and 60 via the tube 71. Further, the driving part 40 includes a pump 101 and a gas tank 33 connected to the pump 101 and containing a gas therein. The gas tank 33 is connected to the closed containers 50 and 60 via a tube 72. The gas contained in the gas tank 33 may be, for example, CO₂, and may be another gas or a mixed gas composed of a plurality of types of gases. In FIG. 2, the illustration of some of the tubes and the pumps is omitted.

### (Culture Circuit)

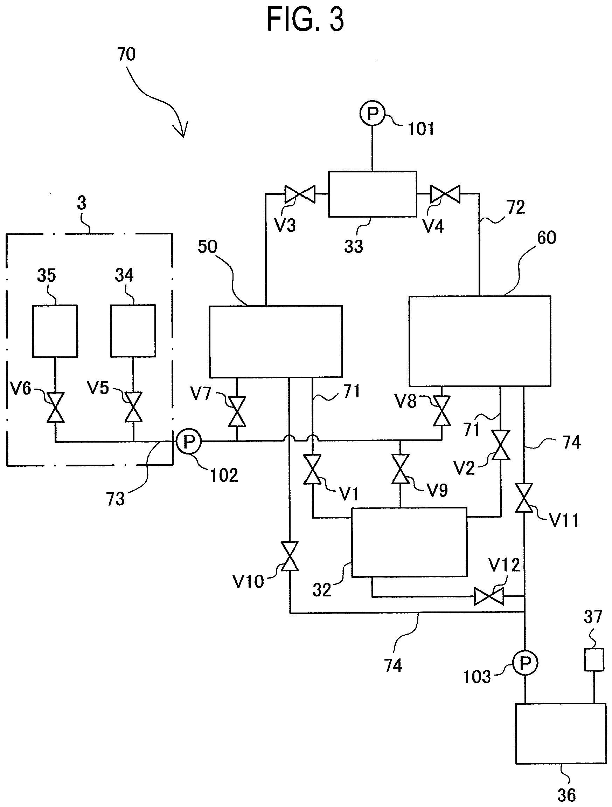

FIG. 3 shows a closed culture circuit 70 that enables cells to be cultured inside the closed containers 50 and 60 while maintaining an aseptic state inside the closed containers 50 and 60. The culture circuit 70 is provided with a medium container 34, a peeling liquid container 35, a waste liquid container 36, and the like, in addition to the closed containers 50 and 60, the stirring part 32 and the gas tank 33 already described above. These parts are connected by tubes 71 to 74. Hereinafter, a detailed description will be given.

The stirring part 32 is connected to the closed containers 50 and 60 via the tube 71. A valve VI is arranged on the tube 71 between the closed container 50 and the stirring part 32, and a valve V2 is arranged on the tube 71 between the closed container 60 and the stirring part 32. Further, the gas tank 33 is connected to the closed containers 50 and 60 via the tube 72. A valve V3 is arranged on the tube 72 between the closed container 50 and the gas tank 33, and a valve V4 is arranged on the tube 72 between the closed container 60 and the gas tank 33.

The medium container 34 and the peeling liquid container 35 are arranged inside the heater 3. The medium container 34 contains a liquid medium for culturing cells. The peeling liquid container 35 contains a peeling liquid for peeling the cells adhering to the inner surfaces of the closed containers 50 and 60. Although not shown, the medium container 34 is connected to a medium tank arranged inside the refrigerating storage part 2 via a tube, and the medium is appropriately supplied from the medium tank to the medium container 34. Similarly, the peeling liquid container 35 is connected to a peeling liquid tank arranged inside the refrigerating storage part 2 via a tube, and the medium is appropriately supplied from the peeling liquid tank to the peeling liquid container 35.

The medium container 34 and the peeling liquid container 35 are connected to the closed containers 50 and 60 and the stirring part 32 via the tube 73. A pump 102 is arranged in the portion of the tube 73 between the medium container 34 and the peeling liquid container 35 and the closed containers 50 and 60 and the stirring part 32. Valves V5 to V9 are arranged on the tube 73. The valve V5 is arranged between the medium container 34 and the pump 102 to switch the supply state of the medium from the medium container 34. The valve V6 is arranged between the peeling liquid container 35 and the pump 102 to switch the supply state of the peeling liquid from the peeling liquid container 35. The valve V7 is arranged between the closed container 50 and the pump 102 to switch the supply state of the medium or the peeling liquid to the closed container 50. The valve V8 is arranged between the closed container 60 and the pump 102 to switch the supply state of the medium or the peeling liquid to the closed container 60. The valve V9 is arranged between the stirring part 32 and the pump 102 to switch the supply state of the medium or the peeling liquid to the stirring part 32.

The waste liquid container 36 is a container to which the waste liquid is discharged from the closed containers 50 and 60 and the stirring part 32. The waste liquid container 36 is formed with a degassing part 37 that communicates with the outside air. The gas inside the waste liquid container 36 is released to the atmosphere through the degassing part 37. A check valve, a filter or the like may be attached to the degassing part 37, if necessary.

The waste liquid container 36 is connected to the closed containers 50 and 60 and the stirring part 32 via the tube 74. Valves V10 to V12 are arranged on the tube 74. The valve V10 is arranged between the closed container 50 and the pump 103 to switch the discharge state of the waste liquid from the closed container 50. The valve V11 is arranged between the closed container 60 and the pump 103 to switch the discharge state of the waste liquid from the closed container 60. The valve V12 is arranged between the stirring part 32 and the pump 103 to switch the discharge state of the waste liquid from the stirring part 32. If the waste liquid from the closed containers 50 and 60 and the stirring part 32 reaches the waste liquid container 36 under gravity, the pump 103 may be omitted.

### (Cell Recovery Operation)

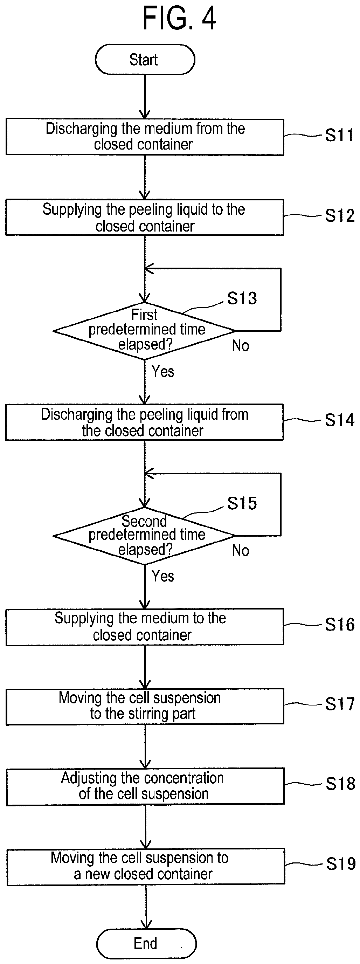

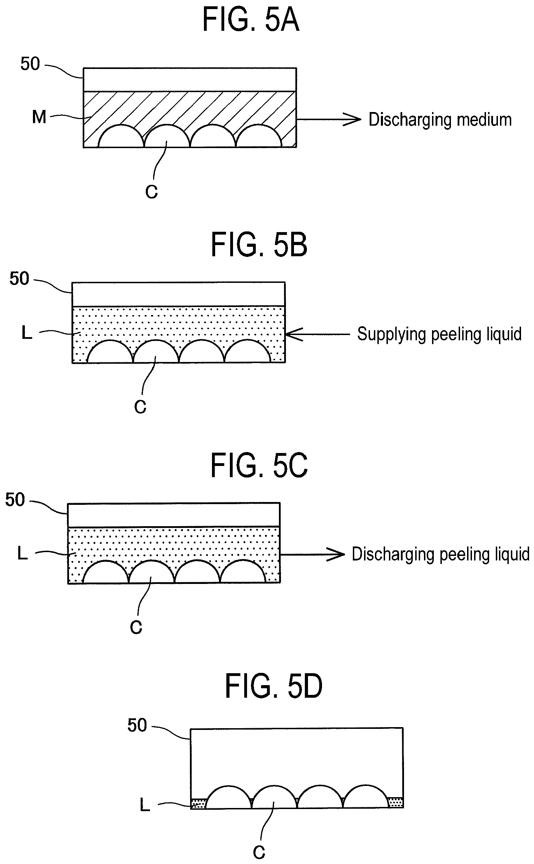

As an example of the cell recovery method according to the present disclosure, the operation at the time of subculture in which the cells cultured in the closed container 50 are transferred to the closed container 60 will be described with reference to FIGS. 4 and 5A to 5E. FIG. 4 is a flowchart showing a series of flows of subculture, and FIGS. 5A to 5E are schematic diagrams showing a cell recovery operation. In the present embodiment, the controller 5 automatically controls the driving of the pumps 101 to 103 and the opening/closing of the valves VI to V12 to automatically perform subculture. Various conditions related to culture and subculture are inputted to the controller 5 in advance by an operator. At the beginning of the subculture, it is assumed that all the valves VI to V12 are closed and the inside of the culture circuit 70 is kept in an aseptic state.

When subculture is performed from the closed container 50 to the closed container 60, first, as shown in FIG. 5A, the medium M is discharged from the closed container 50 (step S11). Specifically, the controller 5 opens the valve V10 and drives the pump 103 to discharge the medium M in the closed container 50 to the waste liquid container 36 via the tube 74. At this time, the cells C adhere to the inner surface of the closed container 50. Therefore, the cells C are not discharged together with the medium M. When the medium M in the closed container 50 is discharged, the controller 5 closes the valve V10 and stops the pump 103.

Next, as shown in FIG. 5B, a peeling liquid L is supplied to the closed container 50 (step S12). Specifically, the controller 5 supplies the peeling liquid L in the peeling liquid container 35 to the closed container 50 via the tube 73 by opening the valves V6 and V7 and driving the pump 102. When a predetermined amount of the peeling liquid L is supplied to the closed container 50, the controller 5 closes the valves V6 and V7 and stops the pump 102.

After the supply of the peeling liquid L to the closed container 50 is completed, the process waits for a first predetermined time in that state (step S13). The first predetermined time is the time until the cells C adhering to the inner surface of the closed container 50 come into a state immediately before being completely peeled by the chemical action of the peeling liquid L. The first predetermined time is appropriately determined depending on the type of cells C and the type of peeling liquid L, and may be, for example, about 2 to 3 minutes.

When the first predetermined time has elapsed (step S13: YES), the peeling liquid L is discharged from the closed container 50 as shown in FIG. 5C (step S14). Specifically, the controller 5 opens the valve V10 and drives the pump 103 to discharge the peeling liquid L in the closed container 50 to the waste liquid container 36 via the tube 74. At this time, the cells C adhere to the inner surface of the closed container 50. Therefore, the cells C are not discharged together with the peeling liquid L. If some of the cells C are peeled until the peeling liquid L is discharged, they may be discharged together with the peeling liquid L. However, such cells C are very few, if any. After the peeling liquid L in the closed container 50 is discharged, the controller 5 closes the valve V10 and stops the pump 103.

After the peeling liquid L is discharged from the closed container 50, the process waits for a second predetermined time in that state (step S15). In step S14, the entire amount of the peeling liquid L is basically discharged from the closed container 50. However, in reality, as shown in FIG. 5D, the residual peeling liquid L adhere to the inner surface of the closed container 50 and the cells C due to surface tension. The second predetermined time is the time until the cells C, which remains in a state immediately before being completely peeled through step S13, are completely peeled by the chemical action of the residual peeling liquid L. The second predetermined time is appropriately determined depending on the type of cells C and the type of peeling liquid L and is, for example, about 2 to 3 minutes.

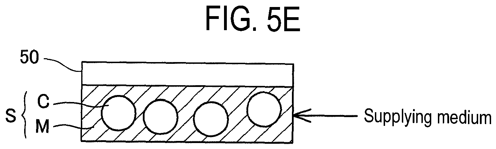

After the second predetermined time has elapsed (step S15: YES), a fresh medium M is supplied to the closed container 50 as shown in FIG. 5E (step S16). Specifically, the controller 5 supplies the medium M in the medium container 34 to the closed container 50 via the tube 73 by opening the valves V5 and V7 and driving the pump 102. Due to step S15, the cells C adhering to the inner surface of the closed container 50 are completely peeled, and the cells C are in a disintegrated state instead of being agglomerated. Therefore, when the liquid medium M is supplied, the cells C in the closed container 50 are mixed with the medium M to form a cell suspension S. Since the residual peeling liquid L is a small amount, the residual peeling liquid L is sufficiently diluted by supplying the medium M and does not pose a problem in a later step. After a predetermined amount of the medium M is supplied to the closed container 50, the controller 5 closes the valves V5 and V7 and stops the pump 102.

Next, the cell suspension S in the closed container 50 is moved to the stirring part 32 (step S17). Specifically, the controller 5 opens the valves VI and V3 and drives the pump 101 to feed the CO₂ in the gas tank 33 to the closed container 50 via the tube 72. As a result, the cell suspension S in the closed container 50 moves to the stirring part 32 through the tube 71. After the cell suspension S is moved to the stirring part 32, the controller 5 closes the valves VI and V3 and stops the pump 101.

Subsequently, the concentration of the cell suspension S in the stirring part 32 is adjusted (step S18). Specifically, a small amount of the cell suspension S whose concentration has become uniform by being stirred by the stirring part 32 is carried to a cell counting part (not shown) where the concentration of the cell suspension S is measured. Based on this measurement result, the controller 5 calculates an additional amount of medium M required to bring the cell suspension S to a predetermined concentration. Then, the controller 5 opens the valves V5 and V9 and drives the pump 102 to supply a predetermined amount of the medium M from the medium container 34 to the stirring part 32. As a result, the concentration of the cell suspension S contained in the stirring part 32 can be adjusted to a predetermined level. After a predetermined amount of the medium M is supplied to the stirring part 32, the controller 5 closes the valves V5 and V9 and stops the pump 102.

Finally, the concentration-adjusted cell suspension S in the stirring part 32 is moved to a new closed container 60 (step S19). Specifically, the controller 5 opens the valves VI to V3 and drives the pump 101 to feed the CO₂ in the gas tank 33 to the stirring part 32 via the closed container 50. As a result, the cell suspension S in the stirring part 32 is moved to the closed container 60 through the tube 71. After the cell suspension S is moved to the closed container 60, the controller 5 closes the valves VI to V3 and stops the pump 101. This completes the subculture. By replacing the closed containers 50 and 60 with new closed containers, it is possible to repeat the subculture.

When the closed containers 50 and 60 are replaced with new closed containers, the tube connected to the closed containers 50 and 60, the stirring part 32 and the like may be replaced with new ones, or the inside of the tube and the stirring part 32 and the like may be replaced with new ones or may be reused after cleaning the inside thereof. When replacing the closed containers 50 and 60, the closed containers 50 and 60 are removed from the tube while maintaining an aseptic state inside the culture circuit 70, and additional new closed containers are connected to the tube while maintaining an aseptic state inside the additional new closed containers. In that case, for example, a welding machine such as BioWelder (manufactured by Sartorius Stedim Japan) or OPTA aseptic connector (manufactured by Sartorius Stedim Japan) may be used.

Steps S11 to S16 in the subculture operation described above correspond to the cell recovery method according to the present disclosure. Specifically, step S1 corresponds to the medium discharge step of the present disclosure, step S12 corresponds to the peeling liquid supply step of the present disclosure, step S14 corresponds to the peeling liquid discharge step of the present disclosure, step S15 corresponds to the waiting step of the present disclosure, and step S16 corresponds to the recovery liquid supply step of the present disclosure. In addition, the medium M is used as the recovery liquid of the present disclosure. Further, the medium supply/discharge device and the recovery liquid supply/discharge device of the present disclosure include the medium container 34, the waste liquid container 36, the tubes 73 and 74, the pumps 102 and 103, the valves V5, V7, V8, V10 and V11. Moreover, the peeling liquid supply/discharge device of the present disclosure includes the peeling liquid container 35, the waste liquid container 36, the tubes 73 and 74, the pumps 102 and 103, the valves V6, V7, V8, V10 and V11.

### (Effect)

In the present embodiment, after the peeling liquid L is discharged, the medium M as the recovery liquid is supplied after waiting until the cells C are peeled by the action of the residual peeling liquid L. Therefore, it is not necessary to separate the cells C from the peeling liquid L, and it is possible to eliminate the need for a centrifuge. Further, since the process waits until the cells C are separated by the action of the residual peeling liquid L, it is not necessary to forcibly separate the cells C adhering to the inner surface of the closed container 50 by the force of the flowing medium M. Moreover, since the cells C are sufficiently separated from each other by the action of the residual peeling liquid L, it is not necessary to disintegrate the cell agglomerates with a tube pump or the like. Therefore, according to the present embodiment, it is possible to suppress damage to the cells C while eliminating the need for a centrifuge.

In the present embodiment, the cell recovery method according to the present disclosure is executed by the controller 5 appropriately controlling the driving of the pumps 101 to 103 and the opening/closing of the valves VI to V12. Therefore, the cell recovery method according to the present disclosure can be automatically executed without human intervention.

In the present embodiment, at least two closed containers 50 and 60 are connected via the connection path 30. By feeding the gas (CO₂) to the closed container 50 after the cell recovery method according to the present disclosure is executed in the closed container 50, the cell suspension S in the closed container 50 is transferred to another closed container 60. According to such a configuration, during the subculture in which the cell suspension S is transferred from the closed container 50 to the closed container 60, the cell suspension S can be transferred without passing through a tube pump or the like. Therefore, it is possible to suppress damage to the cells C.

### (Other Embodiments)

Modifications in which various changes are made to the above-described embodiment will now be described.

In the above-described embodiment, there has been described the example in which the cell recovery method according to the present disclosure is applied to the operation at the time of subculture. Alternatively, the cell recovery method according to the present disclosure may be applied when harvesting cells after the completion of culture.

In the above-described embodiment, the liquid medium is used as the recovery liquid of the present disclosure. However, the type of recovery liquid is not limited thereto. For example, if a frozen liquid is used as the recovery liquid in the cell recovery operation when harvesting cells, the cells can be cryopreserved after the cells are recovered.

In the above-described embodiment, each step of the cell recovery method according to the present disclosure is automatically executed by the controller 5. However, at least a part of the steps may be performed by the operator.

### EXPLANATION OF REFERENCE NUMERALS

1: cell culture device, 5: controller, 30: connection path, 50, 60: closed container (container), C: cell, M: medium (recovery liquid), L: peeling liquid, S: cell suspension

## Claims

1. A cell recovery method for recovering cells which are cultured in at least one container containing a liquid medium and adhere to an inner surface of the container, the method comprising performing:
a medium discharge step of discharging the liquid medium from the container;
after the liquid medium is discharged, a peeling liquid supply step of supplying a peeling liquid for peeling the cells from the inner surface of the container to the container;
a peeling liquid discharge step of discharging the peeling liquid from the container before the cells are completely peeled from the inner surface of the container;
after the peeling liquid is discharged, a waiting step of waiting until the cells are peeled by action of a residual peeling liquid; and
after the waiting step is completed, a recovery liquid supply step of supplying a recovery liquid for recovering the cells to the container.

2. A cell culture device configured to execute the cell recovery method of Claim 1, comprising:
a medium supply/discharge device configured to supply and discharge the liquid medium to and from the container;
a peeling liquid supply/discharge device configured to supply and discharge the peeling liquid to and from the container,
a recovery liquid supply/discharge device configured to supply and discharge the recovery liquid to and from the container; and
a controller,
wherein the cell recovery method is executed by controlling, by the controller, operations of the medium supply/discharge device, the peeling liquid supply/discharge device and the recovery liquid supply/discharge device.

3. The cell culture device of Claim 2, wherein the at least one container includes at least two containers, and the at least two containers are connected via a connection path, and
wherein the recovery liquid containing the cells in one container of the at least two containers is transferred to another container of the at least two containers by feeding a gas to the one container of the at least two containers after the cell recovery method is executed in the one container of the at least two containers.
